# FASCICULE DE BREVET EUROPEEN

(11) **EP 0 773 229 B1**
(45) Date de publication et mention de la délivrance du brevet: **29.10.1997**
(21) Numéro de dépôt: 96402156.2
(22) Date de dépôt: 10.10.1996
(51) Int. Cl.: C08B 37/16, A61K 7/00

(54) **Dérivés de cyclodextrine et leur utilisation, notamment en cosmétique**
Cyclodextrin-Derivate und ihre Verwendung, insbesondere in der Kosmetik
Cyclodextrin derivatives and their use, especially in cosmetics

(30) Priorité: 13.11.1995 FR 9513413
(43) Date de publication de la demande: 14.05.1997
(73) Titulaire: L'OREAL, 75008 Paris (FR)
(72) Inventeur: Richard, Hervé, 93420 Villepinte (FR); Leduc, Madeleine, 75011 Paris (FR)
(74) Mandataire: Dodin, Catherine

(56) Documents cités:
- US-A- 3 565 887

## Description

La présente invention a pour objet de nouveaux composés, dérivés de cyclodextrine, leur procédé de préparation et leur utilisation en cosmétique.

Les cyclodextrines sont des oligosaccharides de formule 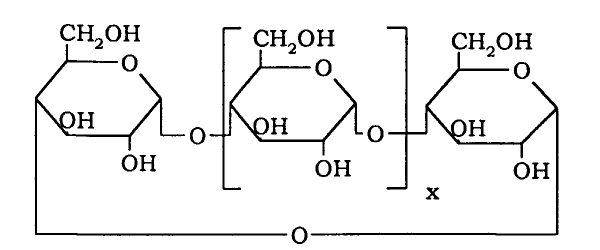 dans laquelle x peut être un nombre égal à 4 (ce qui correspond à l'α-cyclodextrine), à 5 (β-cyclodextrine) ou à 6 (γ-cyclodextrine).
Les cyclodextrines sont des composés bien connus, notamment pour leur aptitude à former des complexes d'inclusion avec des substances actives. Toutefois, ces composés ne sont pas solubles dans les huiles, ce qui limite leur utilisation dans les compositions cosmétiques contenant une phase grasse.

Il est connu de substituer les radicaux hydroxyles des cyclodextrines en vue de modifier les propriétés de ces composés. Ainsi, le brevet US-A-3565887 décrit des esters de cyclodextrines et d'acides gras qui peuvent être utilisés comme agent de surface. Toutefois, la solubilité de ces composés, en particulier la solubilité dans les huiles, reste insuffisante, ce qui limite leur utilisation notamment dans les compositions cosmétiques. Le problème subsiste de disposer de dérivés de cyclodextrine ayant une bonne solubilité dans les huiles.

Après diverses recherches sur les cyclodextrines, la demanderesse a constaté de façon surprenante qu'une nouvelle classe de cyclodextrines permettait de remédier aux inconvénients rencontrés jusqu'à présent.
Elle a constaté que la solubilité dans les huiles de dérivés de cyclodextrine pouvait être améliorée en greffant sur le squelette de base des cyclodextrines des radicaux particuliers.

La présente invention a donc pour objet de nouveaux composés, dérivés de cyclodextrine, répondant à la formule générale (I) suivante :

CD (OH)ₗ (OCOR₁)ₘ (OR₂)ₙ (I)

dans laquelle :
CD représente le squelette de base de l'α-, β- ou γ- cyclodextrine sans les groupements hydroxyle,
OCOR₁ représente un radical, lié au squelette de base de la cyclodextrine, dans lequel R₁ représente un radical alkyle, linéaire ou ramifié, en C₆-C₂₄, ou un radical alcényle, linéaire ou ramifié, en C₈-C₂₄, ou leur mélange,
OR₂ représente un radical, lié au squelette de base de la cyclodextrine, dans lequel R₂ représente un radical alkyle, linéaire ou ramifié, en C₁-C₄ ou un radical hydroxyalkyle en C₂-C₄, l'hydrogène du radical hydroxyle du radical hydroxyalkyle pouvant être substitué par un autre radical hydroxyalkyle en C₂-C₄ ou par un radical -COR₁, ou leur mélange,
m représente le nombre de radicaux OCOR₁ directement liés au squelette de base de la cyclodextrine et est une valeur statistique, différente de 0,
n représente le nombre de radicaux OR₂ directement liés au squelette de base de la cyclodextrine et est une valeur statistique, différente de 0,
I est une valeur statistique telle que (l + m + n) est égal à 18, 21 ou 24 selon que CD représente le squelette de base de l'α-, la β- ou la γ-cyclodextrine, le degré de substitution de la cyclodextrine par les radicaux R₁ allant de 0,2 à 1.

Les nouveaux dérivés de cyclodextrine présentent l'avantage d'avoir une meilleure solubilité dans les huiles que celle des dérivés précédemment connus. Outre la bonne solubilité dans les huiles, les composés selon l'invention possèdent de bonnes propriétés tensioactives permettant leur utilisation dans des compositions cosmétiques, notamment comme agent émulsionnant pour la formulation des émulsions.

Dans le cadre de la présente invention, on entend par degré de substitution de la cyclodextrine par les radicaux R₁, le rapport entre :
. le nombre p de radicaux R₁ provenant, d'une part, des m radicaux (OCOR₁) directement liés au squelette de base de la cyclodextrine et, d'autre part, des éventuels radicaux -COR₁ liés aux radicaux hydroxyalkyle R₂ du radical (OR₂), et
. le nombre (l + m + n) tel que ci-dessus défini, et qui correspond au nombre de radicaux hydroxyle présents dans une cyclodextrine qui serait non substituée. Ainsi, selon que CD représente le squelette de base de l'α-, la β- ou de la γ-cyclodextrine, le degré de substitution est, respectivement, de p/18, p/21 ou p/24.

Selon l'invention, le degré de substitution de la cyclodextrine est de préférence supérieur à 0,4, préférentiellement de l'ordre de 0,45 à 0,95.
La valeur de p peut être déterminée par l'indice de saponification ou par spectre RMN.

De préférence, R₁ désigne un radical alkyle ou alcényle, linéaire ou ramifié, en C₁₁-C₂₁, et en particulier le radical R₁ pris dans le radical -COR₁ peut être choisi parmi les radicaux lauryle, myristyle, palmityle, stéaryle, béhényle, oléyle, linolényle, et leur mélange.

De préférence, R₂ désigne un radical méthyle ou hydroxypropyle, l'hydrogène du radical hydroxypropyle pouvant être substitué par un autre radical hydroxypropyle ou par un radical -COR₁ qui peut être choisi parmi les radicaux lauryle, myristyle, palmityle, stéaryle, béhényle, oléyle, linolényle, et leur mélange. Plus préférentiellement, R₂ désigne un radical hydroxypropyle.

Lorsque CD représente le squelette de base de l'α-cyclodextrine, la somme (l + m + n) est égale à 18, n a de préférence une valeur allant de 3 à 5, et le nombre total p de radicaux R₁ est de préférence compris entre 3,6 et 18.
Lorsque CD représente le squelette de base de la β-cydodextrine, la somme (l + m + n) étant égale à 21, n est de préférence une valeur allant de 3 à 8, préférentiellement de 4 à 7, et en particulier une valeur égale à 4,2 ou 6,3; le nombre total p de radicaux R₁ est de préférence compris entre 4,2 et 21, et plus particulièrement de 10 à 21.
Lorsque CD représente le squelette de base de la γ-cyclodextrine, la somme (l + m + n) étant égale à 24, n est de préférence une valeur allant de 4 à 8, le nombre total p de radicaux R₁ allant de préférence de 4,8 à 24.

De préférence, CD est le squelette de base de la β-cyclodextrine.

La présente invention a également pour objet un procédé de préparation des composés de formule (I).
Ce procédé consiste à faire réagir un dérivé de cyclodextrine de formule CD(OR₂)ₙ(OH)_{l'},
dans laquelle CD, R₂ et n ont la même signification que ci-dessus, I' est une valeur statistique non nulle telle que (l' + n) est égal à 18, 21 ou 24 selon que CD représente le squelette de base de l'α-, la β- ou la γ-cyclodextrine,
avec p moles d'un chlorure d'acide de formule Cl-CO-R₁, p et R₁ ayant la même signification que ci-dessus.

La réaction peut être effectuée dans un solvant organique, notamment anhydre, tel que la pyridine ou le diméthyl formamide.
La réaction peut être effectuée à une température comprise entre 0 °C et 40 °C et de préférence entre 10 °C et 25 °C.
La réaction peut être effectuée en présence d'un catalyseur d'acylation. Celui-ci peut être la para-N,N-diméthylamino pyridine.

La présente invention a également pour objet une composition comprenant un composé de formule (I) tel que défini ci-dessus, ou un mélange de ces composés.

La composition comprenant ledit composé peut se présenter notamment sous forme d'une composition cosmétique, pharmaceutique ou hygiénique comprenant respectivement un milieu cosmétiquement, pharmaceutiquement ou hygiéniquement acceptable.
Dans les compositions selon l'invention, les composés de formule (I) peuvent être présents à une concentration de 0,1 % à 10 % en poids et de préférence de 0,5 % à 5 % en poids par rapport au poids total de la composition.
Les composés selon l'invention peuvent être présents dans la composition sous forme de solution ou de dispersion, aqueuses ou organiques, voire sous forme de dispersion vésiculaire, de dispersion de nanosphères ou de nanocapsules.

Ces compositions peuvent, en outre, comprendre les ingrédients habituellement utilisés dans le domaine considéré. Ainsi elles peuvent contenir au moins un constituant choisi parmi les corps gras, les épaississants, les esters d'acides gras, les esters d'acides gras et de glycérol, les silicones (volatiles ou non, fonctionnalisés ou non), les alcools gras, les tensioactifs, les parfums, les conservateurs, les filtres solaires, les protéines, les vitamines, les polymères, les huiles organiques ou inorganiques, les pigments, les charges, et tout autre additif classiquement utilisé dans les domaines cosmétique, pharmaceutique ou hygiénique.

Ces compositions peuvent être préparées selon les méthodes usuelles connues de l'homme de l'art. Elles peuvent être sous forme de gel, d'émulsion eau-dans-huile ou huile-dans-eau, de stick solide, de spray ou de mousse aérosol.

L'invention a également pour objet l'utilisation des composés de formule (I) tels que définis ci-dessus en tant qu'agent tensioactif, notamment dans le cadre de la formation d'émulsion, de préférence d'émulsion huile-dans-eau ou eau-dans-huile, et plus particulièrement comme agent émulsionnant.
Les composés selon l'invention peuvent également être utilisés comme agent d'encapsulation, notamment d'actifs cosmétiques, pharmaceutiques ou hygiéniques.
Ils peuvent être utilisés dans des compositions de nettoyage de la peau ou des cheveux pour piéger et encapsuler notamment les constituants du sébum de la peau ou du cuir chevelu. En particulier, ils peuvent être utilisés comme agent matifiant de la peau.
L'invention a donc pour objet l'utilisation des composés de formule (I) comme agent d'encapsulation.
Elle a aussi pour objet l'utilisation des composés de formule (I) comme agent de nettoyage de la peau et/ou des cheveux dans une composition cosmétique ou hygiénique.

On va maintenant donner à titre d'illustration et sans aucun caractère limitatif plusieurs exemples de préparation de composés selon l'invention ainsi que des exemples de compositions cosmétiques les comprenant.
Tous les exemples de préparation sont relatifs à des dérivés de β-cyclodextrine. Les exemples 8 à 10 sont des exemples comparatifs et ne font donc pas partie de l'invention.

### Exemple 1 : Préparation d'un composé de formule (I) dans laquelle R₁ est un radical stéaryle, R₂ est un radical hydroxypropyle, n = 6,3, p = 14

A une solution de 12,4 g (8,3 mmole) d'hydroxypropyle β-cyclodextrine (n=6,3); vendue par la société ALDRICH, dans 100 ml de pyridine anhydre, on ajoute à 20°C goutte à goutte pendant 20 minutes 52,5 g (173 mmole) de chlorure de stéaryle. Après 5 heures d'agitation à température ambiante, le mélange réactionnel est versé dans 400 ml d'éthanol. Le solide obtenu est filtré et lavé trois fois à chaud par 200 ml d'éthanol.
Après séchage, on obtient 40,5 g d'un solide beige clair ayant un point de fusion de 39°C.

Infra-rouge : bande ester à 1745 cm⁻¹

### Exemple 2 : Préparation d'un composé de formule (I) dans laquelle R₁ est un radical stéaryle, R₂ est un radical hydroxypropyle, n = 4,2, p = 14

Le composé est préparé selon le mode opératoire de l'exemple 1, en utilisant:
- 6,9 g (5 mmoles) d'hydroxypropyle β-cyclodextrine (n=4,2) vendue sous la dénomination RHODOCAP HP par la société RHÔNE-POULENC
- 31,8 g (105 mmoles) de chlorure de stéaryle

On obtient 28,8 g d'un solide beige clair ayant un point de fusion de 41°C.

Infra-rouge : bande ester à 1745 cm⁻¹

### Exemple 3 : Préparation d'un composé de formule (I) dans laquelle R₁ est un radical oléyle, R₂ est un radical hydroxypropyle, n = 4,2, p = 17

Le composé est préparé selon le mode opératoire de l'exemple 1, en utilisant :
- 6,9 g (5 mmoles) d'hydroxypropyle β-cyclodextrine (n=4,2)
- 31,6 g (105 mmoles) de chlorure d'oléyle

Le milieu réactionnel est versé dans 150 ml d'éthanol. La phase inférieure est extraite et lavée deux fois par 150 ml d'éthanol, puis est reprise dans le dichlorométhane. Après séchage et évaporation de la phase organique, on obtient 24 g d'une huile brune.

Infra-rouge : bande ester à 1745 cm⁻¹

### Exemple 4 : Préparation d'un composé de formule (I) dans laquelle R₁ est un radical palmityle, R₂ est un radical hydroxypropyle, n = 4,2, p = 10

Le composé est préparé selon le mode opératoire de l'exemple 1, en utilisant :
- 6,9 g (5 mmoles) d'hydroxypropyle β-cyclodextrine (n=4,2)
- 13,8 g (50 mmoles) de chlorure de palmityle

On obtient 11,5 g d'un solide transparent blanc ayant un point de ramolissement de 75 °C.

Infra-rouge : bande ester à 1745 cm⁻¹

### Exemple 5 : Préparation d'un dérivé de formule (I) dans laquelle R₁ est un radical palmityle, R₂ est un radical hydroxypropyle, n = 4,2, p = 19

Le composé est préparé selon le mode opératoire de l'exemple 1, en utilisant :
- 6,9 g (5 mmoles) d'hydroxypropyle β-cyclodextrine (n=4,2) auquel on ajoute 4,88 g (40 mmoles) de 4-diméthylamino pyridine, et
- 28,9 g (105 mmoles) de chlorure de palmityle

On laisse sous agitation à température ambiante pendant 17 heures. Puis on ajoute à nouveau goutte à goutte en 20 minutes 28,9 g (105 mmoles) de chlorure de palmityle et on laisse sous agitation à 60 °C pendant 6 heures.
Après traitement du milieu réactionnel, on obtient 29,1 g d'un solide beige clair ayant un point de fusion de 41-43 °C.

Infra-rouge : bande ester à 1745 cm⁻¹

### Exemple 6 : Préparation d'un dérivé de formule (I) dans laquelle R₁ est un radical myristyle, R₂ est un radical hydroxypropyle, n = 4,2, p = 19

Le composé est préparé selon le mode opératoire de l'exemple 1, en utilisant :
- 18,22 g (13,2 mmole) d'hydroxypropyle β-cyclodextrine (n=4,2)
- 68,4 g (277 mmole) de chlorure de myristyle

On obtient 40 g d'une gomme jaune pâle.

Infra-rouge : bande ester à 1745 cm⁻¹

### Exemple 7 : Préparation d'un composé de formule (I) dans laquelle R1 est un radical lauryle, R₂ est un radical hydroxypropyle, n = 4,2, p = 18

Le composé est préparé selon le mode opératoire de l'exemple 1, en utilisant :
- 18,22 g (13,2 mmole) d'hydroxypropyle β-cyclodextrine (n=4,2)
- 60,6 g (277 mmole) de chlorure de lauryle

On obtient 29 g d'une gomme jaune pâle.

Infra-rouge : bande ester à 1745 cm⁻¹

### Exemple 8 : Préparation d'un dérivé de formule (I) dans laquelle R₁ est un radical stéaryle, n = 0, p = 14 (composé qui n'entre pas dans le cadre de l'invention)

Le composé est préparé selon le mode opératoire de l'exemple 1, en utilisant :
- 15 g (13,2 mmoles) de β-cyclodextrine
- 83,9 g (277 mmoles) de chlorure de stéaryle

On laisse sous agitation à température ambiante le mélange hétérogène pendant 18 heures.
Après traitement du milieu réactionnel, on obtient 62,5 g d'un solide blanc cassé ayant un point de fusion de 50 °C.

Infra-rouge : bande ester à 1745 cm⁻¹

### Exemple 9 : Préparation d'un dérivé de formule (I) dans laquelle R₁ est un radical palmityle, n = 0, p = 21 (composé qui n'entre pas dans le cadre de l'invention)

Le composé est préparé selon le mode opératoire de l'exemple 7, en utilisant :
- 5,7 g (5 mmoles) de β-cyclodextrine et 4,88 g (40 mmoles) de 4-diméthylamino pyridine
- 2 x 28,9 g (2 x 105 mmoles) de chlorure de palmityle

Après addition de la première fraction de chlorure d'acide, on laisse sous agitation à température ambiante pendant 23 heures. Après addition de la deuxième fraction de chlorure d'acide, on laisse sous agitation à température ambiante pendant 6 heures.
Après traitement du milieu réactionnel, on obtient 22,5 g d'une cire jaune beige ayant un point de ramolissement de 51 °C.

Infra-rouge : bande ester à 1745 cm⁻¹

### Exemple 10 : Préparation d'un dérivé de formule (I) dans laquelle R1 est un radical stéaryle, R2 est un radical hydroxypropyle, n = 4,2, p = 3 (composé qui n'entre pas dans le cadre de l'invention)

Le composé est préparé selon le mode opératoire de l'exemple 1, en utilisant :
- 1,38 g (1 mmole) d'hydroxypropyle β-cyclodextrine (n=4,2)
- 1,4 g (4,6 mmole) de chlorure de stéaryle

Après purification du brut de réaction par chromatographie sur silice (éluant dichlorométhane/méthanol 95/5), on obtient 1,8 g d'une poudre beige ayant un point de ramolissement de 135 °C.

Infra-rouge : bande ester à 1745 cm⁻¹

### Exemple 11 : Test de solubilité dans les huiles

On a déterminé la solubilité des composés selon l'invention des exemples 1 à 7 et ceux des composés comparatifs 8 à 10 d'une part dans l'huile de vaseline, d'autre part dans le Miglyol 812 de la société DYNAMIT-NOBEL (triglycérides d'acide caprique/caprylique).
La solubilité est déterminée par la quantité maximale de composé qui peut être dissoute dans l'huile testée et est exprimée en pourcentage poids/poids.

On a obtenu les résultats suivants :

| **Exemple** | **R**_{**2**} | **n** | **p** | **p/21** | **Miglyol %** | **Vaseline %** |
|---|---|---|---|---|---|---|
| 1 | HP | 6,3 | 14 | 0,66 | 12 | 12 |
| 2 | HP | 4,2 | 14 | 0,66 | 9 | 7 |
| 3 | HP | 4,2 | 17 | 0,81 | >50 | >50 |
| 4 | HP | 4,2 | 10 | 0,48 | 6 | 8 |
| 5 | HP | 4,2 | 19 | 0,90 | 8 | 12 |
| 6 | HP | 4,2 | 19 | 0,90 | >35 | >20 |
| 7 | HP | 4,2 | 18 | 0,86 | >20 | >30 |
| 8 (c) | - | 0 | 14 | 0,66 | 1 | 1 |
| 9 (c) | - | 0 | 21 | 1 | 1,5 | 2 |
| 10 (c) | HP | 4,2 | 3 | 0,14 | <1 | <1 |
| HP : hydroxypropyle (c) : comparatif | | | | | | |

On constate que seuls les composés conformes à l'invention (exemples 1 à 7) ont une solubilité dans les huiles supérieure à 5 %.
En particulier, les résultats de solubilité des composés des exemples 1, 2 et 8 montrent que pour des composés substitués de manière identique par des radicaux R1 en C17, seuls ceux substitués également par radicaux hydroxypropyle ont une très bonne solubilité dans les huiles.

En comparant la solubilité du composé de l'exemple 10 à celle des composés des exemples 1 à 7, on constate que lorsque le degré de substitution est égal à 0,14, le composé est peu soluble dans les huiles (exemple 10), tandis que lorsque le degré de substitution est de l'ordre de 0,48 ou supérieur, les composés sont bien solubles dans les huiles.

### Exemple 12

On détermine la capacité du composé de l'exemple 1 à modifier la tension interfaciale eau/huile de vaseline par la méthode de l'anneau de LECONTE DU NOUY. Cette méthode consiste à déterminer la force de rappel exercée par une solution du composé de l'exemple 1 sur un anneau qui traverse l'interface.

On a mesuré la tension interfaciale, à 25 °C, entre l'eau et l'huile de vaseline d'une part, et entre l'eau et une solution à 1% du composé de l'exemple 1 dans l'huile de vaseline. Une mesure est effectuée à t₀ juste après la mise en contact des deux phases et à t₁, 1 heure après la mise en contact des deux phases.

On a obtenu les résultats suivants :

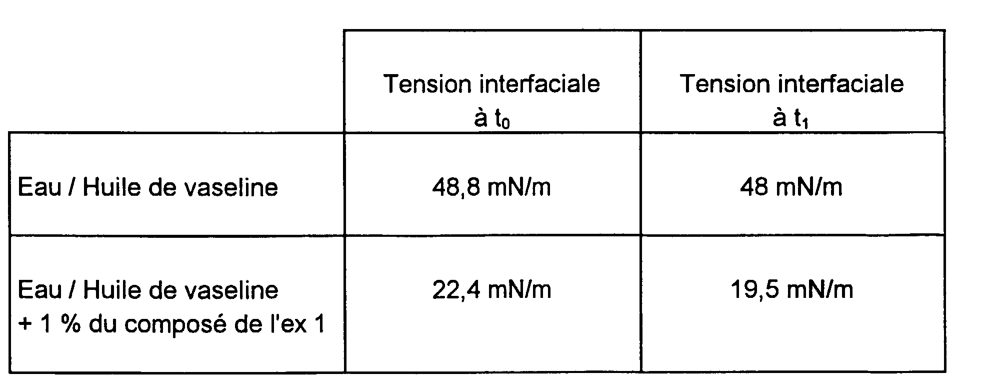

On constate que le composé de l'exemple 1 ajouté à l'huile de vaseline diminue la tension interfaciale eau/huile de vaseline à t₀ et à t₁.
Ce composé présente donc un caractère tensioactif.

### Exemple 13

On prépare une émulsion huile-dans-eau ayant la composition suivante :

| | |
|---|---|
| Composé de l'exemple 1 | 1,5 g |
| Tensioactifs non-ioniques | 5,9 5 g |
| Huile de silicone | 7 g |
| Polyisobutylène hydrogéné | 6 g |
| Agent épaississant | 0,9 g |
| Agent séquestrant | qs |
| Conservateurs | qs |
| Eau | qsp 100 g |

### Exemple 14

On prépare une émulsion ayant la composition suivante :

| | |
|---|---|
| Composé de l'exemple 3 | 1 g |
| Huiles | 20, 5 g |
| Agents émulsionnants | 6, 5 g |
| Acide stéarique | 0, 4 g |
| Glycérine | 3 g |
| Alcool éthylique | 10 g |
| Conservateurs | qs |
| Eau | qsp 100g |

On obtient une crème qui peut être utilisée comme soin antirides du visage.

### Exemple 15

On prépare une émulsion eau-dans-huile ayant la composition suivante :

| | |
|---|---|
| Composé de l'exemple 2 | 1 g |
| Huile de vaseline | 4 g |
| Huile végétale | 6 g |
| Agent émulsionnant | 15 g |
| Glycérine | 5 g |
| Sulfate de magnésium | 1 g |
| Eau | qsp 100 g |

### Exemple 16

On prépare une émulsion huile-dans-eau ayant la composition suivante :

| | |
|---|---|
| Composé de l'exemple 2 | 0,5 g |
| Agent émulsionnant | 3 g |
| Acide stéarique | 0,7 g |
| Huiles | 15 g |
| Stéarate de sucrose | 1,3 g |
| Glycérine | 2 g |
| Hexylène glycol | 4 g |
| Eau | qsp 100 g |

### Exemple 17

On prépare une dispersion de nanoparticules selon le mode opératoire suivant. On a dissous 0,2 g du composé de l'exemple 7 dans 175 ml d'acétone, puis on a ajouté une solution de 0,015 g de β-carotène dissous dans 25 ml d'acétone. On a obtenu ainsi une phase A.
On a préparé également une solution aqueuse contenant 0,25 g de polymère bloc polyoxyéthylène/polyoxypropylène vendu sous la dénomination PLURONIC F68 par la société BASF et 100 g d'eau. Cette solution constitue la phase B. Les phases A et B ont été chauffées à 50 °C, puis la phase A a été versée dans la phase B sous agitation. Le mélange a ensuite été évaporé sous pression réduite jusqu'à un poids de 40 g.
On a obtenu ainsi une dispersion translucide de nanosphères, la taille moyenne des nanosphères étant de 330 nm.

## Revendications

1. Composé, dérivé de cyclodextrine, caractérisé par le fait qu'il répond à la formule générale (I) suivante :
CD (OH)ₗ(OCOR₁)ₘ(OR₂)ₙ (I)
dans laquelle
CD représente le squelette de base de l'α-, β- ou γ- cyclodextrine sans les groupements hydroxyle,
OCOR₁ représente un radical, lié au squelette de base de la cyclodextrine, dans lequel R₁ représente un radical alkyle, linéaire ou ramifié, en C₆-C₂₄, ou un radical alcényle, linéaire ou ramifié, en C₈-C₂₄, ou leur mélange,
OR₂ représente un radical, lié au squelette de base de la cyclodextrine, dans lequel R₂ représente un radical alkyle, linéaire ou ramifié, en C₁-C₄ ou un radical hydroxyalkyle en C₂-C₄, l'hydrogène du radical hydroxyle du radical hydroxyalkyle pouvant être substitué par un autre radical hydroxyalkyle en C₂-C₄ ou par un radical -COR₁, ou leur mélange,
m représente le nombre de radicaux OCOR₁ directement liés au squelette de base de la cyclodextrine et est une valeur statistique, différente de 0,
n représente le nombre de radicaux OR₂ directement liés au squelette de base de la cyclodextrine et est une valeur statistique, différente de 0,
I est une valeur statistique telle que (l + m + n) est égal à 18, 21 ou 24 selon que CD représente le squelette de base de l'α-, la β- ou la γ-cyclodextrine,
le degré de substitution de la cyclodextrine par les radicaux R₁ allant de 0,2 à 1.

2. Composé selon la revendication 1, caractérisé par le fait que R₁ est un radical choisi parmi les radicaux alkyle ou alcényle, linéaires ou ramifiés, en C₁₁-C₂₁

3. Composé selon l'une des revendications précédentes, caractérisé par le fait que R₁ est choisi parmi les radicaux lauryle, myristyle, palmityle, stéaryle, béhényle, oléyle, linolényle, et leur mélange.

4. Composé selon l'une des revendications précédentes, caractérisé par le fait que R₂ désigne un radical méthyle ou hydroxypropyle, l'hydrogène du radical hydroxypropyle pouvant être substitué par un autre radical hydroxypropyle ou par un radical COR₁.

5. Composé selon l'une des revendications précédentes, caractérisé par le fait que CD représente le squelette de base de la β-cyclodextrine.

6. Composé selon l'une des revendications précédentes, caractérisé par le fait que le degré de substitution de la cyclodextrine par les radicaux R₁ est supérieur à 0,4, de préférence compris entre 0,45 et 0,95.

7. Composé selon l'une des revendications précédentes, caractérisé par le fait que CD représente le squelette de base de la β-cyclodextrine et n est compris entre 3 et 8, de préférence 4 à 7.

8. Composé selon la revendication 7, caractérisé par le fait que le nombre total p de radicaux R₁ est compris entre 4,2 et 21, et plus particulièrement de 10 à 21.

9. Procédé de préparation des composés de formule (I), caractérisé par le fait qu'il consiste à faire réagir
(a) un dérivé de cyclodextrine de formule CD(OR₂)ₙ(OH)_{l'}, dans laquelle
CD représente le squelette de base de l'α-, β- ou γ- cyclodextrine sans les groupements hydroxyle,
OR₂ représente un radical, lié au squelette de base de la cyclodextrine, dans lequel R₂ représente un radical alkyle, linéaire ou ramifié, en C₁-C₄ ou un radical hydroxyalkyle en C₂-C₄, l'hydrogène du radical hydroxyle du radical hydroxyalkyle pouvant être substitué par un autre radical hydroxyalkyle en C₂-C₄ ou par un radical -COR₁, ou leur mélange,
n représente le nombre de radicaux OR₂ directement liés au squelette de base de la cyclodextrine et est une valeur statistique, différente de 0,
I' représente une valeur statistique non nulle telle que (l' + n) est égal à 18, 21 ou 24 selon que CD représente le squelette de base de l'α-, la β- ou la γ-cyclodextrine, et
(b) p moles d'un chlorure d'acide de formule Cl-CO-R₁,
R₁ désignant un radical alkyle, linéaire ou ramifié, en C₆-C₂₄, ou un radical alcényle, linéaire ou ramifié, en C₈-C₂₄, ou leur mélange, et
p étant le nombre total de radicaux R₁ .

10. Composition caractérisée par le fait qu'elle comprend un composé de formule (I) tel que défini dans l'une des revendications 1 à 8, ou un mélange de ces composés.

11. Composition selon la revendication 10, caractérisée par le fait qu'elle comprend en outre un milieu cosmétiquement, pharmaceutiquement ou hygiéniquement acceptable.

12. Composition selon l'une des revendications 10 et 11, caractérisée par le fait que le composé de formule (I) est présent à une concentration allant de 0,1 % à 10 % en poids par rapport au poids total de la composition.

13. Composition selon l'une des revendications 10 à 12, caractérisée par le fait que le composé de formule (I) se présente sous forme de solution, de dispersion, de dispersion vésiculaire, de dispersion de nanosphères ou dispersion de nanocapsules.

14. Utilisation d'un composé de formule (I) selon l'une des revendications 1 à 8, comme agent tensioactif.

15. Utilisation d'un composé de formule (I) selon l'une des revendications 1 à 8, comme agent d'encapsulation d'un actif cosmétique, pharmaceutique ou hygiénique.

16. Utilisation d'un composé de formule (I) selon l'une des revendications 1 à 8, comme agent de nettoyage de la peau et/ou des cheveux dans une composition cosmétique ou hygiénique.

## Patentansprüche

1. Von einem Cyclodextrin abgeleitete Verbindungen, dadurch gekennzeichnet, daß sie der folgenden allgemeinen Formel (I) entsprechen:
CD (OH)_{*l*}(OCOR₁)_{*m*}(OR₂)_{*n*} (I),
in der bedeuten:
- CD das α-, β- oder γ-Cyclodextrin-Grundgerüst ohne Hydroxylgruppen,
- OCOR₁ eine an das Cyclodextrin-Grundgerüst gebundene Gruppe, in der R₁ eine geradkettige oder verzweigte C₆₋₂₄-Alkylgruppe oder eine geradkettige oder verzweigte C₈₋₂₄-Alkenylgruppe oder ein Gemisch dieser Gruppen darstellt,
- OR₂ eine an das Cyclodextrin-Grundgerüst gebundene Gruppe, in der R₂ eine geradkettige oder verzweigte C₁₋₄-Alkylgruppe oder eine C₂₋₄-Hydroxyalkylgruppe darstellt, wobei der Hydroxyl-Wasserstoff der Hydroxyalkylgruppe durch eine weitere C₂₋₄-Hydroxyalkylgruppe oder eine Gruppe -COR₁ oder ein Gemisch dieser Gruppen substituiert sein kann,
- *m* die Anzahl der direkt an das Cyclodextrin-Grundgerüst gebundenen Gruppen OCOR₁, die einen statistischen, von 0 verschiedenen Wert darstellt,
- *n* die Anzahl der direkt an das Cyclodextrin-Grundgerüst gebundenen Gruppen OR₂, die einen statistischen, von 0 verschiedenen Wert darstellt
- *l* einen solchen statistischen Wert, daß (*l* + *m* + *n*) gleich 18, 21 oder 24 ist, je nachdem, ob CD das α-, β- oder γ-Cyclodextrin-Grundgerüst darstellt, wobei der Substitutionsgrad des Cyclodextrins bezüglich der R₁-Gruppen 0,2 bis 1 beträgt.

2. Verbindungen nach Anspruch 1, dadurch gekennzeichnet, daß R₁ eine unter geradkettigen und verzweigten C₁₁₋₂₁-Alkyl- und Alkenylgruppen ausgewählte Gruppe ist.

3. Verbindungen nach einem der vorhergehenden Ansprüche, dadurch gekennzeichnet, daß R₁ ausgewählt ist unter den Gruppen Lauryl, Myristyl, Palmityl, Stearyl, Behenyl, Oleyl, Linolenyl sowie Gemischen dieser Gruppen.

4. Verbindungen nach einem der vorhergehenden Ansprüche, dadurch gekennzeichnet, daß R₂ eine Methyl- oder Hydroxypropylgruppe darstellt, wobei der Hydroxyl-Wasserstoff der Hydroxypropylgruppe durch eine weitere Hydroxypropylgruppe oder durch eine Gruppe -COR₁ substituiert sein kann.

5. Verbindungen nach einem der vorhergehenden Ansprüche, dadurch gekennzeichnet, daß CD das β-Cyclodextrin-Grundgerüst darstellt.

6. Verbindungen nach einem der vorhergehenden Ansprüche, dadurch gekennzeichnet, daß der Substitutionsgrad des Cyclodextrins bezüglich der R₁-Gruppen größer als 0,4 ist und vorzugsweise 0,45 bis 0,95 beträgt.

7. Verbindungen nach einem der vorhergehenden Ansprüche, dadurch gekennzeichnet, daß CD das β-Cyclodextrin-Grundgerüst darstellt und *n* 3 bis 8 und vorzugsweise 4 bis 7 beträgt.

8. Verbindungen nach Anspruch 7, dadurch gekennzeichnet, daß die Gesamtzahl *p* der Gruppen R₁ 4,2 bis 21 und insbesondere 10 bis 21 beträgt.

9. Verfahren zur Herstellung der Verbindungen der Formel (I), gekennzeichnet durch Umsetzung
(a) eines Cyclodextrinderivats der Formel CD (OR₂)_{*n*}(OH)_{*l'*}, in der bedeuten:
- CD das α-, β- oder γ-Cyclodextrin-Grundgerüst ohne Hydroxylgruppen,
- OR₂ eine an das Cyclodextrin-Grundgerüst gebundene Gruppe, in der R₂ eine geradkettige oder verzweigte C₁₋₄-Alkylgruppe oder eine C₂₋₄-Hydroxyalkylgruppe darstellt, wobei der Hydroxylwasserstoff der Hydroxyalkylgruppe durch eine weitere C₂₋₄-Hydroxyalkylgruppe oder eine Gruppe -COR₁ oder ein Gemisch dieser Gruppen substituiert sein kann,
- *n* die Anzahl der direkt an das Cyclodextrin-Grundgerüst gebundenen Gruppen OR₂, die einen statistischen, von 0 verschiedenen Werts darstellt,
- *l'* einen solchen statistischen, von 0 verschiedenen Wert, daß (*l'*+ *n*) gleich 18, 21 oder 24 ist, je nachdem, ob CD das α-, β- oder γ-Cyclodextrin-Grundgerüst darstellt,
mit
(b) *p* mol eines Säurechlorids der Formel C1-CO-R₁, in der
- R₁ eine geradkettige oder verzweigte C₆₋₂₄-Alkylgruppe oder eine geradkettige oder verzweigte C₈₋₂₄-Alkenylgruppe oder ein Gemisch dieser Gruppen und
- *p* die Gesamtzahl der Gruppen R₁
darstellen.

10. Zusammensetzung, dadurch gekennzeichnet, daß sie eine Verbindung der Formel (I) nach einem der Ansprüche 1 bis 8 oder ein Gemisch dieser Verbindungen enthält.

11. Zusammensetzung nach Anspruch 10, dadurch gekennzeichnet, daß sie ferner ein kosmetisch, pharmazeutisch oder hygienisch akzeptables Medium enthält.

12. Zusammensetzung nach einem der Ansprüche 10 und 11, dadurch gekennzeichnet, daß die Verbindung der Formel (I) in einer Konzentration von 0,1 bis 10 Gew.-%, bezogen auf das Gesamtgewicht der Zusammensetzung, vorliegt.

13. Zusammensetzung nach einem der Ansprüche 10 bis 12, dadurch gekennzeichnet, daß die Verbindung der Formel (I) in Form einer Lösung, einer Dispersion, einer Vesikeldispersion oder einer Dispersion von Nanokügelchen oder Nanokapseln vorliegt.

14. Verwendung einer Verbindung der Formel (I) nach einem der Ansprüche 1 bis 8 als grenzflächenaktives Mittel.

15. Verwendung einer Verbindung der Formel (I) nach einem der Ansprüche 1 bis 8 als Einkapselungsmittel für einen kosmetischen, pharmazeutischen oder hygienischen Wirkstoff.

16. Verwendung einer Verbindung der Formel (I) nach einem der Ansprüche 1 bis 8 als Reinigungsmittel für die Haut und/oder die Haare in einer kosmetischen oder hygienischen Zusammensetzung.

## Claims

1. Compound, derived from cyclodextrin, characterized in that it corresponds to the following general formula (I):
CD(OH)ₗ(OCOR₁)ₘ(OR₂)ₙ (I)
in which
CD denotes the base skeleton of α-, β- or γ-cyclodextrin without the hydroxyl groups,
OCOR₁ denotes a radical bonded to the base skeleton of cyclodextrin, in which R₁ denotes a C₆-C₂₄ linear or branched alkyl radical or a C₈-C₂₄ linear or branched alkenyl radical or their mixture,
OR₂ denotes a radical bonded to the base skeleton of cyclodextrin, in which R₂ denotes a C₁-C₄ linear or branched alkyl radical or a C₂-C₄ hydroxyalkyl radical, it being possible for the hydrogen of the hydroxyl radical of the hydroxyalkyl radical to be substituted by another C₂-C₄ hydroxyalkyl radical or by a -COR₁ radical, or their mixture,
m denotes the number of OCOR₁ radicals directly bonded to the base skeleton of cyclodextrin and is a statistical value other than 0,
n denotes the number of OR₂ radicals directly bonded to the base skeleton of cyclodextrin and is a statistical value other than 0,
l is a statistical value such that (l + m + n) is equal to 18, 21 or 24, according to whether CD denotes the base skeleton of α-, β- or γ-cyclodextrin,
the degree of substitution of cyclodextrin by the radicals R₁ ranging from 0.2 to 1.

2. Compound according to Claim 1, characterized in that R₁ is a radical chosen from C₁₁-C₂₁ linear or branched alkyl or alkenyl radicals.

3. Compound according to one of the preceding claims, characterized in that R₁ is chosen from lauryl, myristyl, palmityl, stearyl, behenyl, oleyl and linolenyl radicals and their mixture.

4. Compound according to one of the preceding claims, characterized in that R₂ denotes a methyl or hydroxypropyl radical, it being possible for the hydrogen of the hydroxypropyl radical to be substituted by another hydroxypropyl radical or by a radical COR₁.

5. Compound according to one of the preceding claims, characterized in that CD denotes the base skeleton of β-cyclodextrin.

6. Compound according to one of the preceding claims, characterized in that the degree of substitution of cyclodextrin by the radicals R₁ is higher than 0.4, preferably between 0.45 and 0.95.

7. Compound according to one of the preceding claims, characterized in that CD denotes the base skeleton of β-cyclodextrin and n is between 3 and 8, preferably 4 to 7.

8. Compound according to Claim 7, characterized in that the total number p of radicals R₁ is between 4.2 and 21 and more particularly from 10 to 21.

9. Process for the preparation of the compounds of formula (I), characterized in that it consists in reacting
(a) a cyclodextrin derivative of formula CD(OR₂)ₙ(OH)_{l'}, in which
CD denotes the base skeleton of α-, β- or γ-cyclodextrin without the hydroxyl groups,
OR₂ denotes a radical bonded to the base skeleton of cyclodextrin, in which R₂ denotes a C₁-C₄ linear or branched alkyl radical or a C₂-C₄ hydroxyalkyl radical, it being possible for the hydrogen of the hydroxyl radical of the hydroxyalkyl radical to be substituted by another C₂-C₄ hydroxyalkyl radical or by a radical -COR₁, or their mixture,
n denotes the number of OR₂ radicals directly bonded to the base skeleton of cyclodextrin and is a statistical value other than 0,
l' denotes a nonzero statistical value such that (l' + n) is equal to 18, 21 or 24, according to whether CD denotes the base skeleton of α-, β- or γ-cyclodextrin, and
(b) p moles of an acid chloride of formula Cl-CO-R₁,
R₁ denoting a C₆-C₂₄ linear or branched alkyl radical or a C₈-C₂₄ linear or branched alkenyl radical or their mixture, and
p being the total number of radicals R₁.

10. Composition characterized in that it includes a compound of formula (I) as defined in one of Claims 1 to 8 or a mixture of these compounds.

11. Composition according to Claim 10, characterized in that it additionally includes a cosmetically, pharmaceutically or hygienically acceptable medium.

12. Composition according to either of Claims 10 and 11, characterized in that the compound of formula (I) is present in a concentration ranging from 0.1 % to 10 % by weight relative to the total weight of the composition.

13. Composition according to one of Claims 10 to 12, characterized in that the compound of formula (I) is in the form of solution, dispersion, vesicular dispersion, dispersion of nanospheres or dispersion of nanocapsules.

14. Use of a compound of formula (I) according to one of Claims 1 to 8, as surface-active agent.

15. Use of a compound of formula (I) according to one of Claims 1 to 8, as agent for encapsulation of a cosmetic, pharmaceutical or hygienic active substance.

16. Use of a compound of formula (I) according to one of Claims 1 to 8, as agent for cleaning the skin and/or hair in a cosmetic or hygienic composition.
